Europäisches Patentamt

European Patent Office

Office européen des brevets

(19)

(11) Publication number: **0 019 970**
**B1**

(12) **EUROPEAN PATENT SPECIFICATION**

(45) Date of publication of patent specification: **19.10.83**

(51) Int. Cl.³: **A 61 K 7/08**

(21) Application number: **80200466.3**

(22) Date of filing: **19.05.80**

(54) **Conditioning shampoo.**

(30) Priority: **23.05.79 US 41656**

(43) Date of publication of application:
**10.12.80 Bulletin 80/25**

(45) Publication of the grant of the patent:
**19.10.83 Bulletin 83/42**

(84) Designated Contracting States:
**BE DE FR GB IT NL**

(56) References cited:
**CH - A - 490 853**
**DE - B - 1 146 996**
**FR - A - 1 296 934**
**FR - A - 1 568 467**
**US - A - 3 886 277**

(73) Proprietor: **THE PROCTER & GAMBLE COMPANY**
**301 East Sixth Street**
**Cincinnati Ohio 45202 (US)**

(72) Inventor: **Dixon, Thomas Jefferson**
**Hartwood Drive**
**Cincinnati, Ohio 45240 (US)**
Inventor: **Uchtman, Vernon Albert**
**21 Avenell Lane**
**Greenhills, Ohio 45218 (US)**

(74) Representative: **Brooks, Maxim Courtney et al,**
**Procter & Gamble (NTC) Limited Whitley Road**
**Longbenton**
**Newcastle-upon-Tyne NE12 9TS (GB)**

## Conditioning shampoo

The present invention relates to conditioning shampoos which contain a saturated, straight chain fatty acid having from 14 to 18 carbon atoms as the conditioning agent.

Human hair becomes soiled due to its contact with the surrounding atmosphere and, to a greater extent, from sebum secreted by the head. The build-up of sebum causes the hair to have a dirty feel and an unattractive appearance. The soiling of the hair necessitates it being shampooed with frequent regularity.

Shampooing the hair cleans by removing excess soil and sebum. However, the shampooing process has disadvantages in that the hair is left in a wet, tangled, and generally unmanageable state. The latter problem is also prevalent in dry hair. A variety of approaches have been developed to alleviate the after-shampoo problems. These have ranged from the inclusion of hair conditioning aids such as soaps, polymers and cationic agents in shampoos to post-shampoo application of hair conditioners, i.e. hair rinses. Difficulties associated with the use of conditioning aids in shampoos have been compatability problems and a greasy feel on the just-washed hair.

Half rinses typically work by depositing a polymeric film or other material onto the hair. However, such solutions to a very prevalent problem have not been fully satisfactory. For one thing, hair rinses are generally liquid in nature and must be applied in a separate step following the shampooing, left on the hair for a length of time, and rinsed with fresh water. This, of course, is time consuming and is not convenient. The results obtained in this manner also have not been fully satisfactory due to the difficulties associated with the deposition and retention on the hair of the hair conditioning aid. The inclusion of suitable conditioning agents in a shampoo therefore has certain attractive features.

As indicated above, many attempts have been made to incorporate conditioning agents into shampoos. One reference disclosing a conditioning shampoo employing a soap is U.S.—A—3,808,329. The disclosed shampoos have a pH of 7.0 to 8.4 and contain a polyoxyethylene ester to create a milder shampoo. Another reference is U.S.—A—3,590,122, wherein shampoos containing fatty acids which are partially neutralized and having a pH of 5.0 to 8.5 are disclosed. The fatty acids are branched and a preferred acid is isostearic acid.

While the above references disclose using soaps and soaps mixed with unsaponified fatty acids, they are not suggestive of compositions wherein fatty acids are present in a shampoo having a pH of 3.0 to 5.5. Furthermore, they do not suggest the advantages of conditioning with a fatty acid rather than soap.

It is an object of the present invention to provide a superior conditioning shampoo composition.

It is a further object of the present invention to provide a superior hair conditioning shampoo composition comprising a saturated, straight chain fatty acid having from 14 to 18 carbon atoms, an anionic surfactant and having a pH of from 3.0 to 5.5.

A still further object of the present invention is to provide a superior method for cleaning and conditioning hair.

These and other objects of the invention will become apparent from the description to follow.

As used herein, all percentages and ratios are by weight unless otherwise indicated.

Disclosure of invention

The present invention relates to conditioning shampoos containing as the conditioning agent a saturated, straight chain fatty acid having from 14 to 18 carbon atoms. Such compositions comprise:

A. from 10% to 26% of a synthetic anionic surfactant as specified herein;
B. from 1% to 3% of fatty acid selected from myristic acid, palmitic acid, stearic acid and mixtures thereof; and
C. the remainder water

wherein said composition has a pH of from 3.0 to 5.5.

Detailed description of the invention

The conditioning shampoo compositions of the present invention comprise as noted above an anionic surfactant, a fatty acid and water. Suitable surfactants, fatty acids, pH adjustment agents and optional ingredients are discussed in detail below.

Surfactant

One essential component of the conditioning shampoos herein is a surfactant. The term "surfactant" as used herein is intended to denote a synthetic anionic surfactant.

The anionic surfactant herein is selected from the ammonium, sodium, potassium or triethanolamine alkyl sulfates obtained by sulfating the higher alcohols ($C_8$—$C_{18}$ carbon atoms) and the ammonium, triethanolamine, sodium or potassium salts of sulfuric acid esters of the reaction product of 1 mole of a higher fatty alcohol (e.g. tallow or coconut oil alcohols) and 1 to 12 moles of ethylene oxide.

The more preferred surfactants for use in the present compositions include ammonium or sodium lauryl sulfate and ammonium or sodium lauryl ether (3 ethylene oxide groups) sulfate or combinations thereof. Ammonium lauryl sulfate is most preferred as the surfactant for the present compositions. The surfactant level is between 10% and 26%, preferably between 13% and 20%.

### Fatty acid

The conditioning agent of the present composition, a saturated, straight chain fatty acid having from 14 to 18 carbon atoms, is present at a level of from 1% to 3%. Preferably, the level is from 1% to 2%.

The fatty acids are selected from myristic acid, palmitic acid, stearic acid and mixtures thereof. The preferred fatty acid is palmitic acid.

### Aqueous carrier

The shampoos herein are preferably in the form of liquids or creams in which water is the principal diluent. The level of water in the compositions is typically from 35% to 89% by weight.

### pH Adjustment agent

The pH of the shampoo compositions herein lie in the range of 3.0 to 5.5, preferably in the range of 4.5 to 5.0. The pH is kept in the acidic range to maintain the fatty acid in an unsaponified state. Suitable pH adjustment agents include citric acid, phosphoric acid, succinic acid and a sodium citrate/citric acid combination among many others.

### Optional components

The conditioning shampoos herein can contain a variety of non-essential optional ingredients suitable for improving such compositions in a variety of ways. Such conventional optional ingredients are well known to those skilled in the art, e.g., preservatives such as benzyl alcohol, methyl paraben, propyl paraben and imidazolidinylurea; thickeners and viscosity modifiers such as coconut ethanol amide, sodium chloride, ammonium chloride, sodium sulfate, carboxymethyl cellulose, methylcellulose, polyvinyl alcohol, and ethyl alcohol; suspending agents such as magnesium/aluminum silicate; dyes; opacifiers such as behenic acid, ethylene glycol distearate and calcium stearate; sequestering agents such as disodium ethylenediamine tetraacetate; and perfumes. If present, such agents individually generally comprise from 0.01% to 5.0% by weight of the composition.

Surfactants, other than anionics, can also be added as optional components to the present compositions. The optional surfactants may be selected from any of the other types of surfactants but the preferred types are nonionics and amphoterics.

### Method of manufacture

The shampoo compositions of the present invention are made using mixing techniques which are well known in the art. A method of making the present invention is shown in the examples which follow.

### Composition use

In its method aspect, the present invention comprises a method of shampooing the hair by contacting the hair with an amount of the shampoo compositions herein which is effective to clean and condition the hair and rinsing the shampoo from the hair. An effective amount for any individual will depend upon variable factors such as length of the hair, thickness of the hair, amount of soil present, level of surfactant in the shampoo composition, etc. Generally, an effective amount will be from 5 to 40 grams per use.

The following examples will illustrate the invention, but are not intended to be in any way limiting thereof.

### Example I

A typical shampoo composition of the present invention has the following formula:

| Ingredient | Wt.% |
| --- | --- |
| Distilled Water | 80.16 |
| Ammonium Lauryl Sulfate | 15.12 |
| Palmitic Acid | 1.10 |
| Coconut Monoethanolamide | 1.00 |
| Ethylene Glycol Distearate | 1.00 |
| Citric Acid | 0.50 |
| Sodium Hydroxide | 0.30 |
| Ammonium Chloride | 0.25 |
| Fragrance | 0.25 |
| Color (1% Sol) | 0.02 |
| Methyl Paraben | 0.20 |
| Propyl Paraben | 0.10 |
| Total | 100.00 |

### Example II

Another typical composition of the present invention is as follows:

| Ingredient | Wt.% |
| --- | --- |
| Distilled Water | 70.58 |
| Sodium Lauryl Ether (3EO) Sulfate | 25.00 |
| Palmitic Acid | 1.10 |
| Coconut Monoethanolamide | 1.00 |
| Ethylene Glycol Distearate | 1.00 |
| Citric Acid | 0.50 |
| Ammonium Chloride | 0.25 |
| Fragrance | 0.25 |
| Color (1% Sol) | 0.02 |
| Methyl Paraben | 0.20 |
| Propyl Paraben | 0.10 |
| Total | 100.00 |

Freshly washed hair has less of a tendency to "fly away" when combed after drying if the compositions of Examples I and II are used rather than the same compositions without palmitic acid to wash the hair.

Example III

A method which can be used to make the preceding exemplary compositions of the present invention is as follows:

1. The distilled water is heated to 48.8— 54.4°C (120—130°F).

2. The methyl and propyl parabens are slowly added to the water and the mixture is agitated vigorously until both are completely dissolved.

3. The sodium hydroxide is added (if present).

4. The batch is heated until the temperature reaches 54.4°C (130°F) at which temperature the palmitic acid is added. The batch is mixed until the palmitic acid is completely solubilized.

5. The coconut monoethanolamide is added and agitation is maintained at a moderate speed to minimize foaming.

6. The ethylene glycol distearate is added and the batch temperature is allowed to increase to 62.8—65.6°C (145—150°F).

7. Finally the color and the perfume are added and the total batch is allowed to mix for an additional 10 minutes before processing.

## Claims

1. A conditioning shampoo comprising:

(a) from 10% to 26% by weight of a synthetic anionic surfactant selected from ammonium, sodium, potassium and triethanolamine $C_8$— $C_{18}$ alkyl sulfates and alkyl ether sulfates wherein the ether sulfates contain from 1 to 12 moles of ethylene oxide per mole thereof;

(b) from 1% to 3% by weight of a saturated, straight chain fatty acid selected from myristic acid, palmitic acid, stearic acid and mixtures thereof; and

(c) water

characterized in that said composition has a pH of from 3.0 to 5.5.

2. A conditioning shampoo composition according to Claim 1 characterized in that the pH is from 4.5 to 5.0.

3. A conditioning shampoo composition according to Claim 1 or 2 characterized in that it comprises citric acid as pH adjustment agent.

## Patentansprüche

1. Shampoo mit konditionierenden Eigenschaften, umfassend:

(a) 10 Gew.-% bis 26 Gew.-% eines synthetischen anionischen oberflächenaktiven Mittels ausgewählt aus Ammonium-, Natrium-, Kalium- und Triethanolamin-$C_8$— $C_{18}$-alkylsulfaten und -alkylethersulfaten, worin die Ethersulfate 1 bis 12 Mol Ethylenoxid pro Mol davon enthalten;

(b) 1 Gew.-% bis 3 Gew.-% einer gesättigten, geradkettigen Fettsäure ausgewählt aus Myristinsäure, Palmitinsäure, Stearinsäure und Gemischen daraus, und

(c) Wasser,

dadurch gekennzeichnet, daß das Gemisch einen pH-Wert von 3,0 bis 5,5 aufweist.

2. Shampoo mit konditionierenden Eigenschaften gemäß Anspruch 1, dadurch gekennzeichnet, daß der pH-Wert 4,5 bis 5,0 beträgt.

3. Shampoo mit konditionierenden Eigenschaften gemäß Anspruch 1 oder 2, dadurch gekennzeichnet, daß es als Mittel zur Einstellung des pH-Wertes Zitronensäure enthält.

## Revendications

1. Shampooing conditionnant comprenant:

(a) de 10 à 26% en poids d'un surfactif anionique synthétique, choisi parmi des alkyl ($C_8$ à $C_{18}$) sulfates d'ammonium, de sodium, de potassium et de triéthanolamine et des alkyl éther sulfates dans lesquels les éther sulfates contiennent de 1 à 12 moles d'oxyde d'éthylène par mole d'éther;

(b) de 1 à 3% en poids d'un acide gras saturé, à chaîne droite choisi parmi l'acide myristique, l'acide palmitique, l'acide stéarique et leurs mélanges; et

(c) de l'eau,

caractérisé en ce que la composition a un pH compris entre 3,0 et 5,5.

2. Composition de shampooing conditionnant selon la revendication 1, caractérisée en ce que le pH est compris entre 4,5 et 5,0.

3. Composition de shampooing conditionnant selon l'une des revendications 1 et 2, caractérisé en ce qu'elle comprend de l'acide citrique en tant qu'agent régulateur du pH.